(19) Europäisches Patentamt
European Patent Office
Office européen des brevets

(11) Veröffentlichungsnummer : **0 420 091 B1**

(12) # EUROPÄISCHE PATENTSCHRIFT

(45) Veröffentlichungstag der Patentschrift :
**24.11.93 Patentblatt 93/47**

(51) Int. Cl.[5] : **C07C 251/48,** A01N 37/50

(21) Anmeldenummer : **90118274.1**

(22) Anmeldetag : **24.09.90**

(54) **Neue Anilinderivate und diese enthaltende Fungizide.**

Die Akte enthält technische Angaben, die nach dem Eingang der Anmeldung eingereicht wurden und die nicht in dieser Patentschrift enthalten sind.

(30) Priorität : **29.09.89 DE 3932542**

(43) Veröffentlichungstag der Anmeldung :
**03.04.91 Patentblatt 91/14**

(45) Bekanntmachung des Hinweises auf die Patenterteilung :
**24.11.93 Patentblatt 93/47**

(84) Benannte Vertragsstaaten :
**AT BE CH DE DK ES FR GB GR IT LI NL SE**

(56) Entgegenhaltungen :
**EP-A- 0 253 213**
**EP-A- 0 254 426**
**EP-A- 0 278 595**
**EP-A- 0 335 519**

(73) Patentinhaber : **BASF Aktiengesellschaft**
**Carl-Bosch-Strasse 38**
**D-67063 Ludwigshafen (DE)**

(72) Erfinder : **Wenderoth, Bernd, Dr.**
**Schwalbenstrasse 26**
**W-6840 Lampetheim (DE)**
Erfinder : **Schuetz, Franz, Dr.**
**Budapester Strasse 45**
**W-6700 Ludwigshafen (DE)**
Erfinder : **Sauter, Hubert, Dr.**
**Neckarpromenade 20**
**W-6800 Mannheim 1 (DE)**
Erfinder : **Roehl, Franz, Dr.**
**Karl-Otto-Braun-Strasse 8**
**W-6700 Ludwigshafen (DE)**
Erfinder : **Ammermann, Eberhard, Dr.**
**Sachsenstrasse 3**
**W-6700 Ludwigshafen (DE)**
Erfinder : **Lorenz, Gisela, Dr.**
**Erlenweg 13**
**W-6730 Neustadt (DE)**

**EP 0 420 091 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Anilinderivate der allgemeinen Formel I

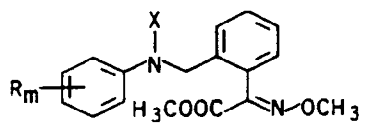

(I)

in der

R

Wasserstoff, Halogen, Cyano, Nitro, $C_1$-$C_{15}$-Alkyl, $C_3$-$C_6$-Cycloalkyl, $C_3$-$C_6$-Alkenyl, $C_1$-$C_4$-Alkoxy, $C_1$-$C_2$-Halogenalkyl, $C_1$-$C_2$-Halogenalkoxy, gegebenenfalls substituiertes Phenyl, gegebenenfalls substituiertes Phenoxy, gegebenenfalls substituiertes Benzyl oder gegebenenfalls substituiertes Benzyloxy,

m

eine ganze Zahl von 1 bis 5 oder die Gruppe

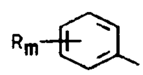

α-Naphthyl oder β-Naphthyl und

X

Wasserstoff, $C_1$-$C_6$-Alkyl oder $C_3$-$C_6$-Cycloalkyl bedeuten, sowie deren pflanzenverträgliche Säureadditionssalze und Metallkomplexverbindungen.

Es ist bekannt, Oximetherderivate, zum Beispiel 2-Phenoxymethyl-phenyl-glyoxylsäuremethylester-O-methyloxim, als Fungizide zu verwenden (EP-A-253213). Ihre Wirkung ist jedoch bei manchen Indikationen nicht ausreichend.

Es ist ferner bekannt, Enoletherderivate, zum Beispiel den 2-(3-Chlorphenyl)-oxymethyl-phenyl-3-methoxy-acrylsäuremethylester als Fungizid zu verwenden (EP-A-278 595).

Der Erfindung lag daher die Aufgabe zugrunde, neue fungizid wirksame Wirkstoffe mit Oximetherstruktur zu finden.

Demgemäß wurden die eingangs definierten Anilinderivate I gefunden. Weitere Gegenstände dieser Erfindung sind ein Verfahren zu Herstellung der Anilinderivate I sowie fungizide Mittel, die die Anilinderivate I als Wirkstoffe enthalten.

Die in der allgemeinen Formel I aufgeführten Reste können beispielsweise folgende Bedeutungen haben:

Die Substituenten R können gleich oder verschieden sein und

Wasserstoff, Halogen, z.B. Fluor, Chlor, Brom, Jod, Cyano, Nitro, $C_1$-$C_{15}$-Alkyl vorzugsweise $C_1$-$C_8$-Alkyl, insbesondere $C_1$-$C_4$-Alkyl, z.B. Methyl, Ethyl, n- oder i-Propyl; n-, i-, s- oder t-Butyl; Pentyl, Hexyl, n-Heptyl, 1,1,3-Trimethyl-butyl, n-Octyl, 1,1,3,3-Tetramethyl-butyl, Nonyl, Decyl, Dodecyl, Tridecyl, Tetradecyl, $C_3$-$C_6$-Cycloalkyl, z.B. Cyclopropyl, Cyclopentyl, Cyclohexyl, $C_3$-$C_6$-Alkenyl, z.B. 1-Propenyl, 2-Propenyl; $C_1$-$C_4$-Alkoxy, z.B. Methoxy, Ethoxy, n-Propoxy, i-Propoxy, n-Butoxy, t-Butoxy, $C_1$-$C_2$-Halogenalkyl, z.B. Difluormethyl, Trifluormethyl, Chlormethyl, Dichlormethyl, Trichlormethyl, Pentafluorethyl, $C_1$-$C_2$-Halogenalkoxy, z.B. Trifluormethoxy, Difluormethoxy, Tetrafluorethoxy, Pentafluorethoxy, gegebenenfalls substituiertes Phenyl, z.B. Phenyl, $C_1$-$C_4$-Alkylphenyl, wie 2-Isopropylphenyl, 2-Methylphenyl, Halogenphenyl wie 2-Chlorphenyl, gegebenenfalls substituiertes Phenoxy, z.B. Phenoxy, $C_1$-$C_4$-Alkylphenoxy wie 2-Methylphenoxy, Halogenphenoxy wie 2-Chlorphenoxy, gegebenenfalls substituiertes Benzyl, z.B. Benzyl, Halogenbenzyl wie 2-Chlorbenzyl, gegebenenfalls substituiertes Benzyloxy, z.B. Benzyloxy, Halogenbenzyloxy, $C_1$-$C_4$-Alkylbenzyloxy wie 2-Chlorbenzyloxy, 2-Methylbenzyloxy, 4-t-Butylbenzyloxy. Die Alkyl, Alkenyl-, Alkoxy- und Halogenalkylreste können unverzweigt oder verzweigt sein. Das gleiche gilt für entsprechende Substituenten der Phenyl-, Phenoxy-, Benzyl- und Benzyloxyreste. Die Zahl der Substituenten der letztgenannten Reste kann zwischen 1 und 3 betragen.

Bevorzugte Reste für R sind Wasserstoff, Halogen, insbesondere Fluor, und $C_1$-$C_4$-Alkyl, insbesondere Methyl.

m

bedeutet 1, 2, 3, 4 oder 5. 1, 2 und 3 werden bevorzugt.

X

bedeutet Wasserstoff, unverzweigtes oder verzweigtes $C_1$-$C_6$-Alkyl, vorzugsweise $C_1$-$C_4$-Alkyl, z.B. Methyl, Ethyl, n-oder i-Propyl, n-, i-, s-oder t-Butyl, Pentyl, Hexyl, oder $C_3$-$C_6$-Cycloalkyl, z.B. Cyclopropyl, Cyclopentyl, Cyclohexyl. X bedeutet insbesondere Methyl.

Die neuen Anilinderivate I können durch Umsetzung mit Säuren auch in pflanzenverträgliche Säureadditionssalze der anorganischen oder organischen Säuren überführt werden, wie beispielsweise in Salze der Salzsäure, Bromwasserstoffsäure, Salpetersäure, Oxalsäure, Essigsäure, Schwefelsäure, Phosphorsäure oder Dodecylbenzolsulfonsäure. Die Wirksamkeit der Salze geht auf das Kation zurück, so daß das Anion im allgemeinen ohne Einfluß ist.

Ferner lassen sich die Verbindungen I nach bekannten Methoden in Metallkomplexe überführen. Das kann durch Umsetzung dieser Verbindungen mit Metallsalzen, z.B. der Metalle Kupfer, Zink, Eisen, Mangan oder Nickel, beispielsweise Kupfer(II)-chlorid, Zink(II)-chlorid, Eisen(III)-chlorid, Kupfer(II)-nitrat, Mangan(II)-chlorid oder Nickel(II)-bromid, erfolgen.

Die Verbindungen I können aufgrund der C=N-Doppelbindungen sowohl als E- als auch als Z-Isomere vorliegen. Sowohl die einzelnen isomeren Verbindungen als auch ihre Gemische werden von der Erfindung umfaßt und sind als fungizide Wirkstoffe brauchbar.

Die Verbindungen I können beispielsweise nach folgendem Verfahren hergestellt werden:

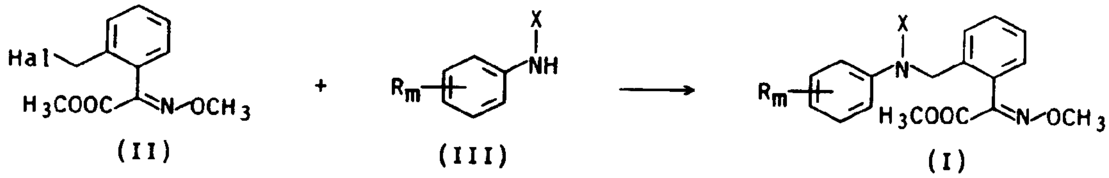

(II) (III) (I)

Ein Benzylhalogenid der allgemeinen Formel II, in der Hal für Chlor, Brom oder Jod steht, wird mit einem Anilin der allgemeinen Formel III, wobei R, m und X die obengenannten Bedeutungen haben, in einem geeigneten organischen Lösungsmittel, zum Beispiel Toluol oder N,N-Dimethylformamid, gegebenenfalls in Gegenwart eines geeigneten Katalysators, wie Kalium- oder Natriumiodid, nach an sich bekannten Verfahren (J. March, Advanced organic Chemistry, 3. Auflage, S. 364-366, J. Wiley & Sons, New York 1985) umgesetzt bei einer Temperatur zwischen 20°C und 120°C, wobei 100 bis 120°C bevorzugt wird.

Die substituierten Benzylhalogenide der allgemeinen Formel II, wobei Hal Chlorid oder Bromid bedeutet, erhält man durch Halogenierung des 2-Methylphenylglyoxylsäuremethylester-O-methyloxims IV nach literaturbekannten Methoden. Dies erreicht man zum Beispiel mit Brom oder Chlor in einem inerten Lösungsmittel (z.B. Tetrachlormethan), gegebenenfalls unter Belichtung mit einer Lichtquelle (z.B. Hg-Dampf-Lampe, 300 W) oder durch Umsetzung mit N-Chlor-bzw. N-Brom-succinimid (Horner, Winkelmann, Angew. Chem. 71 (1959), 349).

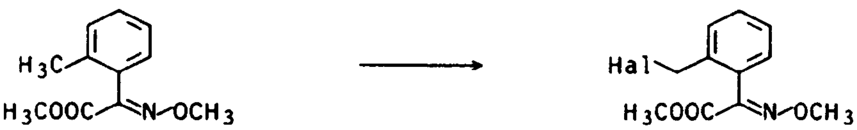

(IV) (II)

Verbindung II mit Hal = Iodid läßt sich nach literaturbekannter Methode aus dem Chlorid oder Bromid durch Umsetzung mit Natriumiodid in Aceton herstellen (Miller, Nunn, J. Chem Soc., Perkin Trans I, 416 (1976). 2-Methylphenylglyoxylsäuremethylester-O-methyloxim IV läßt sich durch Umsetzung von 2-Methylphenylglyoxylsäuremethylester V mit z.B. a) O-Methylhydroxylamin-hydrochlorid oder b) Hydroxylamin-hydrochlorid zum entsprechenden Oxim und Methylierung dieses Oxims mit einem üblichen Methylierungsmittel der Formel $CH_3$-L, in der L eine Abgangsgruppe, z.B. Chlorid, Bromid, Iodid, Methylsulfat, bedeutet (vgl. DE-A-36 23 921).

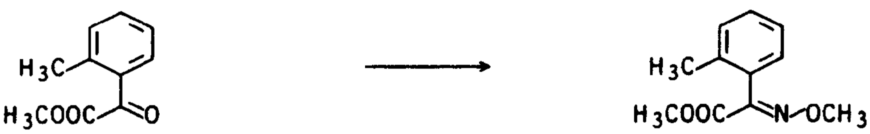

(V) (IV)

Benzylhalogenide der allgemeinen Formel II, wobei Hal Chlorid, Bromid oder Iodid bedeutet, erhält man auch, wenn man 2-Halogenmethyl-phenylglyoxylsäuremethylester VI a) mit O-Methylhydroxylamin-hydrochlorid umsetzt oder b) mit Hydroxylamin-hydrochlorid zum entsprechenden Oxim umsetzt und dieses mit einem Methylierungsmittel der Formel $CH_3$-L, in der L eine Abgangsgruppe, z.B. Chlorid, Bromid, Iodid, Methylsulfat, bedeutet, zur 1 Reaktion bringt (DE-A-36 23 921).

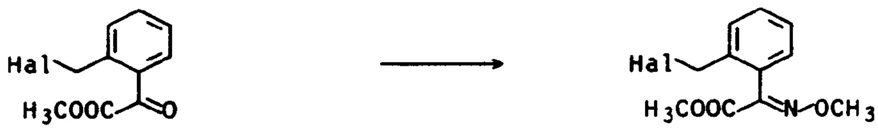

(VI) (II)

2-Halogenmethylphenylglyoxylsäure-methylester der Formel VI, wobei Hal Chlorid oder Bromid bedeutet, lassen sich herstellen, indem man 2-Methylphenylglyoxylsäuremethylester V nach literaturbekannten Methoden halogeniert. Die Umsetzung erfolgt zum Beispiel mit Brom oder Chlor in einem inerten Lösungsmittel (z.B. Tetrachlormethan), gegebenenfalls unter Belichtung mit einer Lichtquelle (z.B. Hg-Dampf-Lampe, 300 W) oder mit N-Chlor- bzw. N-Brom-Succinimid (Horner, Winkelmann, Angew. Chem. 71 (1959), 349).

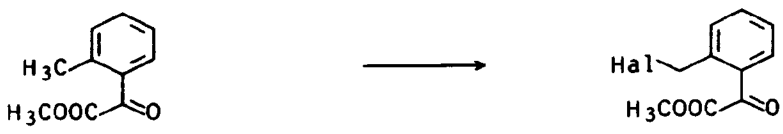

(V) (VI)

Verbindung VI mit Hal = Iodid läßt sich nach literaturbekannter Methode aus dem Chlorid oder Bromid durch Umsetzung mit Natriumiodid in Aceton herstellen (J. Chem. Soc., Perkin Trans I, 416 (1976).

Herstellungsbeispiele

Beispiel 1

a) 27,6 g (0,133 mol) 2-Methyl-phenylglyoxylsäuremethylester-O-methyloxim gelöst in 400 ml Tetrachlormethan werden unter Rühren mit 21,4 g (0,133 mol) Brom versetzt. Dann wird unter Bestrahlung mit einer 300 W-Hg-Dampf-Lampe vier Stunden auf Rückflußtemperatur erhitzt. Danach wird eingeengt, in Essigester/Wasser aufgenommen, mit Wasser gewaschen, mit Natriumsulfat getrocknet und eingeengt. Das Rohprodukt wird mit Cyclohexan/Essigester (9/1) an Kieselgel chromatographisch gereinigt. Es werden 17,4 g (46 % d. Th.) 2-(Brommethyl)-phenylglyoxylsäuremethylester-O-methyloxim als Öl erhalten.

b) 6,4 g (60 mmol) N-Methylanilin, 8,6 g (30 mmol) 2-(Brommethyl)-phenylglyoxylsäuremethylester-O-methyloxim und circa 100 mg Kaliumiodid werden in 100 ml Toluol für 7 Stunden unter Rühren auf Rückflußtemperatur erhitzt. Nach dem Abkühlen wird mehrmals mit Wasser gewaschen, über Natriumsulfat getrocknet und eingeengt. Es werden 9 g (96 % d. Th.) 2-[(N-Methyl-N-phenyl)-aminomethyl]-phenylglyoxylsäuremethylester -O-methyloxim (Tabelle 1, Nr. 1) erhalten (Fp.=90-92°C).

$^1$H-NMR:$\delta$ = 2.95(S,3H); 3.75(S,3H); 4.05(S,3H); 4.35(S,2H); 6.65-7.35(m,9H).

Analog in der J. March, Advanced Organic Chemistry, 3. Auflage, S. 364-366, 1985) und in Beispiel 1 beschriebenen Verfahren können die in Tabelle 1 aufgeführten Wirkstoffe hergestellt werden.

Tabelle 1

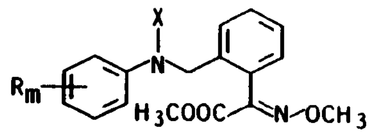

(I)

| Nr. | $R_m$ | X | Fp(°C) / IR($cm^{-1}$) |
|---|---|---|---|
| 1 | H | $CH_3$ | 90-92 / 2935, 1725, 1600, 1507, 1215, 1069, 1017, 751 |
| 2 | 2-F | $CH_3$ | |
| 3 | 3-F | $CH_3$ | 101-102 / 2935, 1723, 1620, 1502, 1214, 1066, 1014, 761 |
| 4 | 4-F | $CH_3$ | 73-75 / 2940, 1725, 1512, 1227, 1069, 1017, 819 |
| 5 | 2,3-$F_2$ | $CH_3$ | |
| 6 | 2,4-$F_2$ | $CH_3$ | |
| 7 | 2,4,6-$F_3$ | $CH_3$ | |
| 8 | 2,3,4,5,6-$F_5$ | $CH_3$ | |
| 9 | 2-Cl | $CH_3$ | |
| 10 | 3-Cl | $CH_3$ | |
| 11 | 4-Cl | $CH_3$ | |
| 12 | 2,3-$Cl_2$ | $CH_3$ | |
| 13 | 2,4-$Cl_2$ | $CH_3$ | |
| 14 | 2,5-$Cl_2$ | $CH_3$ | |
| 15 | 2,6-$Cl_2$ | $CH_3$ | |
| 16 | 3,4-$Cl_2$ | $CH_3$ | |
| 17 | 3,5-$Cl_2$ | $CH_3$ | |
| 18 | 2,3,4-$Cl_3$ | $CH_3$ | |

Tabelle 1 (Fortsetzung)

| Nr. | $R_m$ | X | Fp($^oC$) / IR($cm^{-1}$) |
|---|---|---|---|
| 19 | 2,3,5-$Cl_3$ | $CH_3$ | |
| 20 | 2,3,6-$Cl_3$ | $CH_3$ | |
| 21 | 2,4,5-$Cl_3$ | $CH_3$ | |
| 22 | 2,4,6-$Cl_3$ | $CH_3$ | |
| 23 | 3,4,5-$Cl_3$ | $CH_3$ | |
| 24 | 2,3,4,6-$Cl_4$ | $CH_3$ | |
| 25 | 2,3,5,6-$Cl_4$ | $CH_3$ | |
| 26 | 2,3,4,5,6-$Cl_5$ | $CH_3$ | |
| 27 | 2-Br | $CH_3$ | |
| 28 | 3-Br | $CH_3$ | |
| 29 | 4-Br | $CH_3$ | |
| 30 | 2,4-$Br_2$ | $CH_3$ | |
| 31 | 2,5-$Br_2$ | $CH_3$ | |
| 32 | 2,6-$Br_2$ | $CH_3$ | |
| 33 | 2,4,6-$Br_3$ | $CH_3$ | |
| 34 | 2,3,4,5,6-$Br_5$ | $CH_3$ | |
| 35 | 2-J | $CH_3$ | |
| 36 | 3-J | $CH_3$ | |
| 37 | 4-J | $CH_3$ | |
| 38 | 2,4-$J_2$ | $CH_3$ | |
| 39 | 2-Cl, 3-F | $CH_3$ | |
| 40 | 2-Cl, 4-F | $CH_3$ | |
| 41 | 2-Cl, 5-F | $CH_3$ | |

Tabelle 1 (Fortsetzung)

| Nr. | $R_m$ | X | Fp($^oC$) / IR($cm^{-1}$) |
|---|---|---|---|
| 42 | 2-Cl, 6-F | $CH_3$ | |
| 43 | 2-Cl, 3-Br | $CH_3$ | |
| 44 | 2-Cl, 4-Br | $CH_3$ | |
| 45 | 2-Cl, 5-Br | $CH_3$ | |
| 46 | 2-Cl, 6-Br | $CH_3$ | |
| 47 | 2-Br, 3-Cl | $CH_3$ | |
| 48 | 2-Br, 4-Cl | $CH_3$ | |
| 49 | 2-Br, 5-Cl | $CH_3$ | |
| 50 | 2-Br, 3-F | $CH_3$ | |
| 51 | 2-Br, 4-F | $CH_3$ | |
| 52 | 2-Br, 5-F | $CH_3$ | |
| 53 | 2-Br, 6-F | $CH_3$ | |
| 54 | 2-F, 3-Cl | $CH_3$ | |
| 55 | 2-F, 4-Cl | $CH_3$ | |
| 56 | 2-F, 5-Cl | $CH_3$ | |
| 57 | 3-Cl, 4-F | $CH_3$ | |
| 58 | 3-Cl, 5-F | $CH_3$ | |
| 59 | 3-Cl, 4-Br | $CH_3$ | |
| 60 | 3-Cl, 5-Br | $CH_3$ | |
| 61 | 3-F, 4-Cl | $CH_3$ | |
| 62 | 3-F, 4-Br | $CH_3$ | |
| 63 | 3-Br, 4-Cl | $CH_3$ | |
| 64 | 3-Br, 4-F | $CH_3$ | |

Tabelle 1 (Fortsetzung)

| Nr. | $R_m$ | X | Fp(°C) / IR($cm^{-1}$) |
|---|---|---|---|
| 65 | 2,6-$Cl_2$, 4-Br | $CH_3$ | |
| 66 | 2-$CH_3$ | $CH_3$ | |
| 67 | 3-$CH_3$ | $CH_3$ | 68-72 / 2935,1755,1603,1213,1068,1017,767 |
| 68 | 4-$CH_3$ | $CH_3$ | 80-82 / 2940,1726,1522,1321,1215,1068,1018,805 |
| 69 | 2,3-$(CH_3)_2$ | $CH_3$ | |
| 70 | 2,4-$(CH_3)_2$ | $CH_3$ | |
| 71 | 2,5-$(CH_3)_2$ | $CH_3$ | |
| 72 | 2,6-$(CH_3)_2$ | $CH_3$ | |
| 73 | 3,4-$(CH_3)_2$ | $CH_3$ | |
| 74 | 3,5-$(CH_3)_2$ | $CH_3$ | |
| 75 | 2,3,4-$(CH_3)_3$ | $CH_3$ | |
| 76 | 2,3,5-$(CH_3)_3$ | $CH_3$ | |
| 77 | 2,3,6-$(CH_3)_3$ | $CH_3$ | |
| 78 | 2,4,5-$(CH_3)_3$ | $CH_3$ | |
| 79 | 2,4,6-$(CH_3)_3$ | $CH_3$ | |
| 80 | 3,4,5-$(CH_3)_3$ | $CH_3$ | |
| 81 | 2,3,4,6-$(CH_3)_4$ | $CH_3$ | |
| 82 | 2,3,5,6-$(CH_3)_4$ | $CH_3$ | |
| 83 | 2,3,4,5,6-$(CH_3)_5$ | $CH_3$ | |
| 84 | 2-$C_2H_5$ | $CH_3$ | |
| 85 | 3-$C_2H_5$ | $CH_3$ | |
| 86 | 4-$C_2H_5$ | $CH_3$ | |
| 87 | 2,4-$(C_2H_5)_2$ | $CH_3$ | |

Tabelle 1 (Fortsetzung)

| Nr. | $R_m$ | X | Fp($^oC$) / IR($cm^{-1}$) |
|---|---|---|---|
| 88 | 2,6-$(C_2H_5)_2$ | $CH_3$ | |
| 89 | 3,5-$(C_2H_5)_2$ | $CH_3$ | |
| 90 | 2,4,6-$(C_2H_5)_3$ | $CH_3$ | |
| 91 | 2-n-$C_3H_7$ | $CH_3$ | |
| 92 | 3-n-$C_3H_7$ | $CH_3$ | |
| 93 | 4-n-$C_3H_7$ | $CH_3$ | |
| 94 | 2-i-$C_3H_7$ | $CH_3$ | |
| 95 | 3-i-$C_3H_7$ | $CH_3$ | |
| 96 | 4-i-$C_3H_7$ | $CH_3$ | |
| 97 | 2,4-(i-$C_3H_7)_2$ | $CH_3$ | |
| 98 | 2,6-(i-$C_3H_7)_2$ | $CH_3$ | |
| 99 | 3,5-(i-$C_3H_7)_2$ | $CH_3$ | |
| 100 | 2,4,6-(i-$C_3H_7)_3$ | $CH_3$ | |
| 101 | 2-s-$C_4H_9$ | $CH_3$ | |
| 102 | 3-s-$C_4H_9$ | $CH_3$ | |
| 103 | 4-s-$C_4H_9$ | $CH_3$ | |
| 104 | 2-t-$C_4H_9$ | $CH_3$ | |
| 105 | 3-t-$C_4H_9$ | $CH_3$ | |
| 106 | 4-t-$C_4H_9$ | $CH_3$ | |
| 107 | 2,3-(t-$C_4H_9)_2$ | $CH_3$ | |
| 108 | 2,4-(t-$C_4H_9)_2$ | $CH_3$ | |
| 109 | 2,5-(t-$C_4H_9)_2$ | $CH_3$ | |
| 110 | 2,6-(t-$C_4H_9)_2$ | $CH_3$ | |

Tabelle 1 (Fortsetzung)

| Nr. | $R_m$ | X | Fp(°C) / IR($cm^{-1}$) |
|---|---|---|---|
| 111 | 3,5-(t-$C_4H_9$)$_2$ | $CH_3$ | |
| 112 | 2,4,6-(t-$C_4H_9$)$_3$ | $CH_3$ | |
| 113 | 4-n-$C_9H_{19}$ | $CH_3$ | |
| 114 | 4-n-$C_{12}H_{25}$ | $CH_3$ | |
| 115 | 3-n-$C_{15}H_{31}$ | $CH_3$ | |
| 116 | 4-(1,1,3,3,-Tetramethylbutyl) | $CH_3$ | |
| 117 | 4-(1,1,3,-Trimethylbutyl) | $CH_3$ | |
| 118 | 2-t-$C_4H_9$, 4-$CH_3$ | $CH_3$ | |
| 119 | 2-t-$C_4H_9$, 5-$CH_3$ | $CH_3$ | |
| 120 | 2,6-(t-$C_4H_9$)$_2$, 4-$CH_3$ | $CH_3$ | |
| 121 | 2-$CH_3$, 4-t-$C_4H_9$ | $CH_3$ | |
| 122 | 2-$CH_3$, 6-t-$C_4H_9$ | $CH_3$ | |
| 123 | 2-$CH_3$, 4-i-$C_3H_7$ | $CH_3$ | |
| 124 | 2-$CH_3$, 5-i-$C_3H_7$ | $CH_3$ | |
| 125 | 3-$CH_3$, 4-i-$C_3H_7$ | $CH_3$ | |
| 126 | 2-i-$C_3H_7$, 5-$CH_3$ | $CH_3$ | |
| 127 | 2,4-(t-$C_4H_9$)$_2$, 6-i-$C_3H_7$ | $CH_3$ | |
| 128 | 2-Allyl ($C_3H_5$) | $CH_3$ | |
| 129 | 3-Allyl | $CH_3$ | |
| 130 | 4-Allyl | $CH_3$ | |
| 131 | 2-$C_3H_5$, 6-$CH_3$ | $CH_3$ | |
| 132 | 2-cyclo-$C_6H_{11}$ | $CH_3$ | |
| 133 | 3-cyclo-$C_6H_{11}$ | $CH_3$ | |

Tabelle 1 (Fortsetzung)

| Nr. | $R_m$ | X | Fp(°C) / IR($cm^{-1}$) |
|---|---|---|---|
| 134 | 4-cyclo-$C_6H_{11}$ | $CH_3$ | |
| 135 | 2,4-(cyclo-$C_6H_{11}$)$_2$, 6-$CH_3$ | $CH_3$ | |
| 136 | 2-$CH_3$, 4-cyclo-$C_6H_{11}$ | $CH_3$ | |
| 137 | 2-$CH_3$, 4-(1,1,3,3-Tetramethylbutyl) | $CH_3$ | |
| 138 | 2-$CH_2C_6H_5$ | $CH_3$ | |
| 139 | 3-$CH_2C_6H_5$ | $CH_3$ | |
| 140 | 4-$CH_2C_6H_5$ | $CH_3$ | |
| 141 | 2-$CH_2C_6H_5$, 4-$CH_3$ | $CH_3$ | |
| 142 | 2-$CH_3$, 4-$CH_2C_6H_5$ | $CH_3$ | |
| 143 | 2-$C_6H_5$ | $CH_3$ | |
| 144 | 3-$C_6H_5$ | $CH_3$ | |
| 145 | 4-$C_6H_5$ | $CH_3$ | |
| 146 | 4-(2-i-$C_3H_7$-$C_6H_4$) | $CH_3$ | |
| 147 | 4-$C_6H_5$, 2,6-($CH_3$)$_2$ | $CH_3$ | |
| 148 | 2-Cl, 4-$C_6H_5$ | $CH_3$ | |
| 149 | 2-Br, 4-$C_6H_5$ | $CH_3$ | |
| 150 | 2-$C_6H_5$, 4-Cl | $CH_3$ | |
| 151 | 2-$C_6H_5$, 4-Br | $CH_3$ | |
| 152 | 2-$CH_2C_6H_5$, 4-Cl | $CH_3$ | |
| 153 | 2-$CH_2C_6H_5$, 4-Br | $CH_3$ | |
| 154 | 2-Cl, 4-$CH_2C_6H_5$ | $CH_3$ | |
| 155 | 2-Br, 4-$CH_2C_6H_5$ | $CH_3$ | |
| 156 | 2-cyclo-$C_6H_{11}$, 4-Cl | $CH_3$ | |

Tabelle 1 (Fortsetzung)

| Nr. | $R_m$ | X | Fp(°C) / IR($cm^{-1}$) |
|---|---|---|---|
| 157 | 2-cyclo-$C_6H_{11}$, 4-Br | $CH_3$ | |
| 158 | 2-Cl, 4-cyclo-$C_6H_{11}$ | $CH_3$ | |
| 159 | 2-Br, 4-cyclo-$C_6H_{11}$ | $CH_3$ | |
| 160 | 2-$OCH_3$ | $CH_3$ | |
| 161 | 3-$OCH_3$ | $CH_3$ | |
| 162 | 4-$OCH_3$ | $CH_3$ | |
| 163 | 2,4-$(OCH_3)_2$ | $CH_3$ | |
| 164 | 2-$OC_2H_5$ | $CH_3$ | |
| 165 | 3-$OC_2H_5$ | $CH_3$ | |
| 166 | 4-$OC_2H_5$ | $CH_3$ | |
| 167 | 2-$OCH_2C_6H_5$ | $CH_3$ | |
| 168 | 3-$OCH_2C_6H_5$ | $CH_3$ | |
| 169 | 4-$OCH_2C_6H_5$ | $CH_3$ | |
| 170 | 2-O-t-$C_4H_9$ | $CH_3$ | |
| 171 | 3-O-t-$C_4H_9$ | $CH_3$ | |
| 172 | 4-O-t-$C_4H_9$ | $CH_3$ | |
| 173 | 2-$OC_6H_5$ | $CH_3$ | |
| 174 | 3-$OC_6H_5$ | $CH_3$ | |
| 175 | 4-$OC_6H_5$ | $CH_3$ | |
| 176 | 2-$CF_3$ | $CH_3$ | |
| 177 | 3-$CF_3$ | $CH_3$ | |
| 178 | 4-$CF_3$ | $CH_3$ | |
| 179 | 2-$OCF_3$ | $CH_3$ | |

Tabelle 1 (Fortsetzung)

| Nr. | $R_m$ | X | Fp(°C) / IR($cm^{-1}$) |
|---|---|---|---|
| 180 | 3-$OCF_3$ | $CH_3$ | |
| 181 | 4-$OCF_3$ | $CH_3$ | |
| 182 | 3-$OCH_2CHF_2$ | $CH_3$ | |
| 183 | 3-$OCF_2CHF_2$ | $CH_3$ | |
| 184 | 3-$OC_2F_5$ | $CH_3$ | |
| 185 | 2-$NO_2$ | $CH_3$ | |
| 186 | 3-$NO_2$ | $CH_3$ | |
| 187 | 4-$NO_2$ | $CH_3$ | |
| 188 | 2-CN | $CH_3$ | |
| 189 | 3-CN | $CH_3$ | |
| 190 | 4-CN | $CH_3$ | |
| 191 | 2-$CH_3$, 3-Cl | $CH_3$ | |
| 192 | 2-$CH_3$, 4-Cl | $CH_3$ | |
| 193 | 2-$CH_3$, 5-Cl | $CH_3$ | |
| 194 | 2-$CH_3$, 6-Cl | $CH_3$ | |
| 195 | 2-$CH_3$, 3-F | $CH_3$ | |
| 196 | 2-$CH_3$, 4-F | $CH_3$ | |
| 197 | 2-$CH_3$, 5-F | $CH_3$ | |
| 198 | 2-$CH_3$, 6-F | $CH_3$ | |
| 199 | 2-$CH_3$, 3-Br | $CH_3$ | |
| 200 | 2-$CH_3$, 4-Br | $CH_3$ | |
| 201 | 2-$CH_3$, 5-Br | $CH_3$ | |
| 202 | 2-$CH_3$, 6-Br | $CH_3$ | |

Tabelle 1 (Fortsetzung)

| Nr. | $R_m$ | X | Fp($^oC$) / IR($cm^{-1}$) |
|---|---|---|---|
| 203 | 2-Cl, 3-$CH_3$ | $CH_3$ | |
| 204 | 2-Cl, 4-$CH_3$ | $CH_3$ | |
| 205 | 2-Cl, 5-$CH_3$ | $CH_3$ | |
| 206 | 2-F, 3-$CH_3$ | $CH_3$ | |
| 207 | 2-F, 4-$CH_3$ | $CH_3$ | |
| 208 | 2-F, 5-$CH_3$ | $CH_3$ | |
| 209 | 2-Br, 3-$CH_3$ | $CH_3$ | |
| 210 | 2-Br, 4-$CH_3$ | $CH_3$ | |
| 211 | 2-Br, 5-$CH_3$ | $CH_3$ | |
| 212 | 3-$CH_3$, 4-Cl | $CH_3$ | |
| 213 | 3-$CH_3$, 5-Cl | $CH_3$ | |
| 214 | 3-$CH_3$, 4-F | $CH_3$ | |
| 215 | 3-$CH_3$, 5-F | $CH_3$ | |
| 216 | 3-$CH_3$, 4-Br | $CH_3$ | |
| 217 | 3-$CH_3$, 5-Br | $CH_3$ | |
| 218 | 3-F, 4-$CH_3$ | $CH_3$ | |
| 219 | 3-Cl, 4-$CH_3$ | $CH_3$ | |
| 220 | 3-Br, 4-$CH_3$ | $CH_3$ | |
| 221 | 2-Cl, 4,5-$(CH_3)_2$ | $CH_3$ | |
| 222 | 2-Br, 4,5-$(CH_3)_2$ | $CH_3$ | |
| 223 | 2-Cl, 3,5-$(CH_3)_2$ | $CH_3$ | |
| 224 | 2-Br, 3,5-$(CH_3)_2$ | $CH_3$ | |
| 225 | 2,6-$Cl_2$, 4-$CH_3$ | $CH_3$ | |
| 226 | 2,6-$F_2$, 4-$CH_3$ | $CH_3$ | |

Tabelle 1 (Fortsetzung)

| Nr. | $R_m$ | X | Fp(°C) / IR($cm^{-1}$) |
|---|---|---|---|
| 227 | 2,6-$Br_2$, 4-$CH_3$ | $CH_3$ | |
| 228 | 2,4-$Cl_2$, 6-$CH_3$ | $CH_3$ | |
| 229 | 2,4-$F_2$, 6-$CH_3$ | $CH_3$ | |
| 230 | 2,4-$Br_2$, 6-$CH_3$ | $CH_3$ | |
| 231 | 2,6-$(CH_3)_2$, 4-F | $CH_3$ | |
| 232 | 2,6-$(CH_3)_2$, 4-Cl | $CH_3$ | |
| 233 | 2,6-$(CH_3)_2$, 4-Br | $CH_3$ | |
| 234 | 3,5-$(CH_3)_2$, 4-F | $CH_3$ | |
| 235 | 3,5-$(CH_3)_2$, 4-Cl | $CH_3$ | |
| 236 | 3,5-$(CH_3)_2$, 4-Br | $CH_3$ | |
| 237 | 2,3,6-$(CH_3)_3$, 4-F | $CH_3$ | |
| 238 | 2,3,6-$(CH_3)_3$, 4-Cl | $CH_3$ | |
| 239 | 2,3,6-$(CH_3)_3$, 4-Br | $CH_3$ | |
| 240 | 2,4-$(CH_3)_2$, 6-F | $CH_3$ | |
| 241 | 2,4-$(CH_3)_2$, 6-Cl | $CH_3$ | |
| 242 | 2,4-$(CH_3)_2$, 6-Br | $CH_3$ | |
| 243 | 2-i-$C_3H_7$, 4-Cl, 5-$CH_3$ | $CH_3$ | |
| 244 | 2-$CH_3$, 4-Cl, 5-i-$C_3H_7$ | $CH_3$ | |
| 245 | 2-Cl, 3-i-$C_3H_7$ | $CH_3$ | |
| 246 | 2-Cl, 4-i-$C_3H_7$ | $CH_3$ | |
| 247 | 2-Cl, 4-$NO_2$ | $CH_3$ | |
| 248 | 2-$NO_2$, 4-Cl | $CH_3$ | |
| 249 | 2-$OCH_3$, 5-$NO_2$ | $CH_3$ | |

Tabelle 1 (Fortsetzung)

| Nr. | $R_m$ | X | Fp(°C) / IR($cm^{-1}$) |
|---|---|---|---|
| 250 | 2,4-$Cl_2$, 5-$NO_2$ | $CH_3$ | |
| 251 | 2,4-$Cl_2$, 6-$NO_2$ | $CH_3$ | |
| 252 | 2,6-$Cl_2$, 4-$NO_2$ | $CH_3$ | |
| 253 | 2,6-$Br_2$, 4-$NO_2$ | $CH_3$ | |
| 254 | 2,6-$J_2$, 4-$NO_2$ | $CH_3$ | |
| 255 | H | $C_2H_5$ | |
| 256 | 2-F | $C_2H_5$ | |
| 257 | 3-F | $C_2H_5$ | |
| 258 | 4-F | $C_2H_5$ | |
| 259 | 2,3-$F_2$ | $C_2H_5$ | |
| 260 | 2,4-$F_2$ | $C_2H_5$ | |
| 261 | 2,4,6-$F_3$ | $C_2H_5$ | |
| 262 | 2,3,4,5,6-$F_5$ | $C_2H_5$ | |
| 263 | 2-Cl | $C_2H_5$ | |
| 264 | 3-Cl | $C_2H_5$ | |
| 265 | 4-Cl | $C_2H_5$ | |
| 266 | 2,3-$Cl_2$ | $C_2H_5$ | |
| 267 | 2,4-$Cl_2$ | $C_2H_5$ | |
| 268 | 2,5-$Cl_2$ | $C_2H_5$ | |
| 269 | 2,6-$Cl_2$ | $C_2H_5$ | |
| 270 | 3,4-$Cl_2$ | $C_2H_5$ | |
| 271 | 3,5-$Cl_2$ | $C_2H_5$ | |
| 272 | 2,3,4-$Cl_3$ | $C_2H_5$ | |

Tabelle 1 (Fortsetzung)

| Nr. | $R_m$ | X | Fp(°C) / IR($cm^{-1}$) |
|---|---|---|---|
| 273 | 2,3,5-$Cl_3$ | $C_2H_5$ | |
| 274 | 2,3,6-$Cl_3$ | $C_2H_5$ | |
| 275 | 2,4,5-$Cl_3$ | $C_2H_5$ | |
| 276 | 2,4,6-$Cl_3$ | $C_2H_5$ | |
| 277 | 3,4,5-$Cl_3$ | $C_2H_5$ | |
| 278 | 2,3,4,6-$Cl_4$ | $C_2H_5$ | |
| 279 | 2,3,5,6-$Cl_4$ | $C_2H_5$ | |
| 280 | 2,3,4,5,6-$Cl_5$ | $C_2H_5$ | |
| 281 | 2-Br | $C_2H_5$ | |
| 282 | 3-Br | $C_2H_5$ | |
| 283 | 4-Br | $C_2H_5$ | |
| 284 | 2,4-$Br_2$ | $C_2H_5$ | |
| 285 | 2,5-$Br_2$ | $C_2H_5$ | |
| 286 | 2,6-$Br_2$ | $C_2H_5$ | |
| 287 | 2,4,6-$Br_3$ | $C_2H_5$ | |
| 288 | 2,3,4,5,6-$Br_5$ | $C_2H_5$ | |
| 289 | 2-J | $C_2H_5$ | |
| 290 | 3-J | $C_2H_5$ | |
| 291 | 4-J | $C_2H_5$ | |
| 292 | 2,4-$J_2$ | $C_2H_5$ | |
| 293 | 2-Cl, 3-F | $C_2H_5$ | |
| 294 | 2-Cl, 4-F | $C_2H_5$ | |
| 295 | 2-Cl, 5-F | $C_2H_5$ | |

Tabelle 1 (Fortsetzung)

| Nr. | $R_m$ | X | Fp(°C) / IR($cm^{-1}$) |
|---|---|---|---|
| 296 | 2-Cl, 3-F | $C_2H_5$ | |
| 297 | 2-Cl, 3-Br | $C_2H_5$ | |
| 298 | 2-Cl, 4-Br | $C_2H_5$ | |
| 299 | 2-Cl, 5-Br | $C_2H_5$ | |
| 300 | 2-Cl, 6-Br | $C_2H_5$ | |
| 301 | 2-Br, 3-Cl | $C_2H_5$ | |
| 302 | 2-Br, 4-Cl | $C_2H_5$ | |
| 303 | 2-Br, 5-Cl | $C_2H_5$ | |
| 304 | 2-Br, 3-F | $C_2H_5$ | |
| 305 | 2-Br, 4-F | $C_2H_5$ | |
| 306 | 2-Br, 5-F | $C_2H_5$ | |
| 307 | 2-Br, 6-F | $C_2H_5$ | |
| 308 | 2-F, 3-Cl | $C_2H_5$ | |
| 309 | 2-F, 4-Cl | $C_2H_5$ | |
| 310 | 2-F, 5-Cl | $C_2H_5$ | |
| 311 | 3-Cl, 4-F | $C_2H_5$ | |
| 312 | 3-Cl, 5-F | $C_2H_5$ | |
| 313 | 3-Cl, 4-Br | $C_2H_5$ | |
| 314 | 3-Cl, 5-Br | $C_2H_5$ | |
| 315 | 3-F, 4-Cl | $C_2H_5$ | |
| 316 | 3-F, 4-Br | $C_2H_5$ | |
| 317 | 3-Br, 4-Cl | $C_2H_5$ | |
| 318 | 3-Br, 4-F | $C_2H_5$ | |

Tabelle 1 (Fortsetzung)

| Nr. | $R_m$ | X | Fp($^oC$) / IR($cm^{-1}$) |
|---|---|---|---|
| 319 | 2,6-$Cl_2$, 4-Br | $C_2H_5$ | |
| 320 | 2-$CH_3$ | $C_2H_5$ | |
| 321 | 3-$CH_3$ | $C_2H_5$ | |
| 322 | 4-$CH_3$ | $C_2H_5$ | |
| 323 | 2,3-$(CH_3)_2$ | $C_2H_5$ | |
| 324 | 2,4-$(CH_3)_2$ | $C_2H_5$ | |
| 325 | 2,5-$(CH_3)_2$ | $C_2H_5$ | |
| 326 | 2,6-$(CH_3)_2$ | $C_2H_5$ | |
| 327 | 3,4-$(CH_3)_2$ | $C_2H_5$ | |
| 328 | 3,5-$(CH_3)_2$ | $C_2H_5$ | |
| 329 | 2,3,4-$(CH_3)_3$ | $C_2H_5$ | |
| 330 | 2,3,5-$(CH_3)_3$ | $C_2H_5$ | |
| 331 | 2,3,6-$(CH_3)_3$ | $C_2H_5$ | |
| 332 | 2,4,5-$(CH_3)_3$ | $C_2H_5$ | |
| 333 | 2,4,6-$(CH_3)_3$ | $C_2H_5$ | |
| 334 | 3,4,5-$(CH_3)_3$ | $C_2H_5$ | |
| 335 | 2,3,4,6-$(CH_3)_4$ | $C_2H_5$ | |
| 336 | 2,3,4,6-$(CH_3)_4$ | $C_2H_5$ | |
| 337 | 2,3,4,5,6-$(CH_3)_5$ | $C_2H_5$ | |
| 338 | 2-$C_2H_5$ | $C_2H_5$ | |
| 339 | 3-$C_2H_5$ | $C_2H_5$ | |
| 340 | 4-$C_2H_5$ | $C_2H_5$ | |
| 341 | 2,4-$(C_2H_5)_2$ | $C_2H_5$ | |

Tabelle 1 (Fortsetzung)

| Nr. | $R_m$ | X | Fp($^oC$) / IR($cm^{-1}$) |
|---|---|---|---|
| 342 | 2,6-$(C_2H_5)_2$ | $C_2H_5$ | |
| 343 | 3,5-$(C_2H_5)_2$ | $C_2H_5$ | |
| 344 | 2,4,6-$(C_2H_5)_3$ | $C_2H_5$ | |
| 345 | 2-n-$C_3H_7$ | $C_2H_5$ | |
| 346 | 3-n-$C_3H_7$ | $C_2H_5$ | |
| 347 | 4-n-$C_3H_7$ | $C_2H_5$ | |
| 348 | 2-i-$C_3H_7$ | $C_2H_5$ | |
| 349 | 3-i-$C_3H_7$ | $C_2H_5$ | |
| 350 | 4-i-$C_3H_7$ | $C_2H_5$ | |
| 351 | 2,4-(i-$C_3H_7$)$_2$ | $C_2H_5$ | |
| 352 | 2,6-(i-$C_3H_7$)$_2$ | $C_2H_5$ | |
| 353 | 2,4,6-(i-$C_3H_7$)$_3$ | $C_2H_5$ | |
| 354 | 2,4,6-(i-$C_3H_7$)$_3$ | $C_2H_5$ | |
| 355 | 2-s-$C_4H_9$ | $C_2H_5$ | |
| 356 | 3-s-$C_4H_9$ | $C_2H_5$ | |
| 357 | 4-s-$C_4H_9$ | $C_2H_5$ | |
| 358 | 2-t-$C_4H_9$ | $C_2H_5$ | |
| 359 | 3-t-$C_4H_9$ | $C_2H_5$ | |
| 360 | 4-t-$C_4H_9$ | $C_2H_5$ | |
| 361 | 2,3-(t-$C_4H_9$)$_2$ | $C_2H_5$ | |
| 362 | 2,4-(t-$C_4H_9$)$_2$ | $C_2H_5$ | |
| 363 | 2,5-(t-$C_4H_9$)$_2$ | $C_2H_5$ | |
| 364 | 2,6-(t-$C_4H_9$)$_2$ | $C_2H_5$ | |

Tabelle 1 (Fortsetzung)

| Nr. | $R_m$ | X | Fp(°C) / IR($cm^{-1}$) |
|---|---|---|---|
| 365 | 3,5-(t-$C_4H_9$)$_2$ | $C_2H_5$ | |
| 366 | 2,4,6-(t-$C_4H_9$)$_3$ | $C_2H_5$ | |
| 367 | 4-n-$C_9H_{19}$ | $C_2H_5$ | |
| 368 | 4-n-$C_{12}H_{25}$ | $C_2H_5$ | |
| 369 | 3-n-$C_{15}H_{31}$ | $C_2H_5$ | |
| 370 | 4-(1,1,3,3,-Tetramethylbutyl) | $C_2H_5$ | |
| 371 | 4-(1,1,3,-Trimethylbutyl) | $C_2H_5$ | |
| 372 | 2-t-$C_4H_9$, 4-$CH_3$ | $C_2H_5$ | |
| 373 | 2-t-$C_4H_9$, 5-$CH_3$ | $C_2H_5$ | |
| 374 | 2,6-(t-$C_4H_9$)$_2$, 4-$CH_3$ | $C_2H_5$ | |
| 375 | 2-$CH_3$, 4-t-$C_4H_9$ | $C_2H_5$ | |
| 376 | 2-$CH_3$, 6-t-$C_4H_9$ | $C_2H_5$ | |
| 377 | 2-$CH_3$, 4-i-$C_3H_7$ | $C_2H_5$ | |
| 378 | 2-$CH_3$, 5-i-$C_3H_7$ | $C_2H_5$ | |
| 379 | 3-$CH_3$, 4-i-$C_3H_7$ | $C_2H_5$ | |
| 380 | 2-i-$C_3H_7$, 5-$CH_3$ | $C_2H_5$ | |
| 381 | 2,4-(t-$C_4H_9$)$_2$, 6-i-$C_3H_7$ | [illegible] | |
| 382 | 2-Allyl ($C_3H_5$) | $C_2H_5$ | |
| 383 | 3-Allyl | $C_2H_5$ | |
| 384 | 4-Allyl | $C_2H_5$ | |
| 385 | 2-$C_3H_5$, 6-$CH_3$ | $C_2H_5$ | |
| 386 | 2-cyclo-$C_6H_{11}$ | $C_2H_5$ | |
| 387 | 3-cyclo-$C_6H_{11}$ | $C_2H_5$ | |

Tabelle 1 (Fortsetzung)

| Nr. | $R_m$ | X | Fp($^oC$) / IR($cm^{-1}$) |
|---|---|---|---|
| 388 | 4-cyclo-$C_6H_{11}$ | $C_2H_5$ | |
| 389 | 2,4-(cyclo-$C_6H_{11}$)$_2$, 6-$CH_3$ | $C_2H_5$ | |
| 390 | 2-$CH_3$, 4-cyclo-$C_6H_{11}$ | $C_2H_5$ | |
| 391 | 2-$CH_3$, 4-(1,1,3,3-Tetramethylbutyl) | $C_2H_5$ | |
| 392 | 2-$CH_2C_6H_5$ | $C_2H_5$ | |
| 393 | 3-$CH_2C_6H_5$ | $C_2H_5$ | |
| 394 | 4-$CH_2C_6H_5$ | $C_2H_5$ | |
| 395 | 2-$CH_2C_6H_5$, 4-$CH_3$ | $C_2H_5$ | |
| 396 | 2-$CH_3$, 4-$CH_2C_6H_5$ | $C_2H_5$ | |
| 397 | 2-$C_6H_5$ | $C_2H_5$ | |
| 398 | 3-$C_6H_5$ | $C_2H_5$ | |
| 399 | 4-$C_6H_5$ | $C_2H_5$ | |
| 400 | 4-(2-i-$C_3H_7$-$C_6H_4$) | $C_2H_5$ | |
| 401 | 4-$C_6H_5$, 2,6-($CH_3$)$_2$ | $C_2H_5$ | |
| 402 | 2-Cl, 4-$C_6H_5$ | $C_2H_5$ | |
| 403 | 2-Br, 4-$C_6H_5$ | $C_2H_5$ | |
| 404 | 2-$C_6H_5$, 4-Cl | $C_2H_5$ | |
| 405 | 2-$C_6H_5$, 4-Br | $C_2H_5$ | |
| 406 | 2-$CH_2C_6H_5$, 4-Cl | $C_2H_5$ | |
| 407 | 2-$CH_2C_6H_5$, 4-Br | $C_2H_5$ | |
| 408 | 2-Cl, 4-$CH_2C_6H_5$ | $C_2H_5$ | |
| 409 | 2-Br, 4-$CH_2C_6H_5$ | $C_2H_5$ | |
| 410 | 2-cyclo-$C_6H_{11}$, 4-Cl | $C_2H_5$ | |

Tabelle 1 (Fortsetzung)

| Nr. | $R_m$ | X | Fp(°C) / IR($cm^{-1}$) |
|---|---|---|---|
| 411 | 2-cyclo-$C_6H_{11}$, 4-Br | $C_2H_5$ | |
| 412 | 2-Cl, 4-cyclo-$C_6H_{11}$ | $C_2H_5$ | |
| 413 | 2-Br, 4-cyclo-$C_6H_{11}$ | $C_2H_5$ | |
| 414 | 2-$OCH_3$ | $C_2H_5$ | |
| 415 | 3-$OCH_3$ | $C_2H_5$ | |
| 416 | 4-$OCH_3$ | $C_2H_5$ | |
| 417 | 2,4-$(OCH_3)_2$ | $C_2H_5$ | |
| 418 | 2-$OC_2H_5$ | $C_2H_5$ | |
| 419 | 3-$OC_2H_5$ | $C_2H_5$ | |
| 420 | 4-$OC_2H_5$ | $C_2H_5$ | |
| 421 | 2-$OCH_2C_6H_5$ | $C_2H_5$ | |
| 422 | 3-$OCH_2C_6H_5$ | $C_2H_5$ | |
| 423 | 4-$OCH_2C_6H_5$ | $C_2H_5$ | |
| 424 | 2-O-t-$C_4H_9$ | $C_2H_5$ | |
| 425 | 3-O-t-$C_4H_9$ | $C_2H_5$ | |
| 426 | 4-O-t-$C_4H_9$ | $C_2H_5$ | |
| 427 | 2-$OC_6H_5$ | $C_2H_5$ | |
| 428 | 3-$OC_6H_5$ | $C_2H_5$ | |
| 429 | 4-$OC_6H_5$ | $C_2H_5$ | |
| 430 | 2-$CF_3$ | $C_2H_5$ | |
| 431 | 3-$CF_3$ | $C_2H_5$ | |
| 432 | 4-$CF_3$ | $C_2H_5$ | |
| 433 | 2-$OCF_3$ | $C_2H_5$ | |

Tabelle 1 (Fortsetzung)

| Nr. | $R_m$ | X | Fp($^oC$) / IR($cm^{-1}$) |
|---|---|---|---|
| 434 | 3-$OCF_3$ | $C_2H_5$ | |
| 435 | 4-$OCF_3$ | $C_2H_5$ | |
| 436 | 3-$OCH_2CHF_2$ | $C_2H_5$ | |
| 437 | 3-$OCF_2CHF_2$ | $C_2H_5$ | |
| 438 | 3-$OC_2F_5$ | $C_2H_5$ | |
| 439 | 2-$NO_2$ | $C_2H_5$ | |
| 440 | 3-$NO_2$ | $C_2H_5$ | |
| 441 | 4-$NO_2$ | $C_2H_5$ | |
| 442 | 2-CN | $C_2H_5$ | |
| 443 | 3-CN | $C_2H_5$ | |
| 444 | 4-CN | $C_2H_5$ | |
| 445 | 2-$CH_3$, 3-Cl | $C_2H_5$ | |
| 446 | 2-$CH_3$, 4-Cl | $C_2H_5$ | |
| 447 | 2-$CH_3$, 5-Cl | $C_2H_5$ | |
| 448 | 2-$CH_3$, 6-Cl | $C_2H_5$ | |
| 449 | 2-$CH_3$, 3-F | $C_2H_5$ | |
| 450 | 2-$CH_3$, 4-F | $C_2H_5$ | |
| 451 | 2-$CH_3$, 5-F | $C_2H_5$ | |
| 452 | 2-$CH_3$, 6-F | $C_2H_5$ | |
| 453 | 2-$CH_3$, 3-Br | $C_2H_5$ | |
| 454 | 2-$CH_3$, 4-Br | $C_2H_5$ | |
| 455 | 2-$CH_3$, 5-Br | $C_2H_5$ | |
| 456 | 2-$CH_3$, 6-Br | $C_2H_5$ | |

Tabelle 1 (Fortsetzung)

| Nr. | $R_m$ | X | Fp($^oC$) / IR($cm^{-1}$) |
|---|---|---|---|
| 457 | 2-Cl, 3-$CH_3$ | $C_2H_5$ | |
| 458 | 2-Cl, 4-$CH_3$ | $C_2H_5$ | |
| 459 | 2-Cl, 5-$CH_3$ | $C_2H_5$ | |
| 460 | 2-F, 3-$CH_3$ | $C_2H_5$ | |
| 461 | 2-F, 4-$CH_3$ | $C_2H_5$ | |
| 462 | 2-F, 5-$CH_3$ | $C_2H_5$ | |
| 463 | 2-Br, 3-$CH_3$ | $C_2H_5$ | |
| 464 | 2-Br, 4-$CH_3$ | $C_2H_5$ | |
| 465 | 2-Br, 5-$CH_3$ | $C_2H_5$ | |
| 466 | 3-$CH_3$, 4-Cl | $C_2H_5$ | |
| 467 | 3-$CH_3$, 5-Cl | $C_2H_5$ | |
| 468 | 3-$CH_3$, 4-F | $C_2H_5$ | |
| 469 | 3-$CH_3$, 5-F | $C_2H_5$ | |
| 470 | 3-$CH_3$, 4-Br | $C_2H_5$ | |
| 471 | 3-$CH_3$, 5-Br | $C_2H_5$ | |
| 472 | 3-F, 4-$CH_3$ | $C_2H_5$ | |
| 473 | 3-Cl, 4-$CH_3$ | $C_2H_5$ | |
| 474 | 3-Br, 4-$CH_3$ | $C_2H_5$ | |
| 475 | 2-Cl, 4,5-$(CH_3)_2$ | $C_2H_5$ | |
| 476 | 2-Br, 4,5-$(CH_3)_2$ | $C_2H_5$ | |
| 477 | 2-Cl, 3,5-$(CH_3)_2$ | $C_2H_5$ | |
| 478 | 2-Br, 3,5-$(CH_3)_2$ | $C_2H_5$ | |
| 479 | 2,6-$Cl_2$, 4-$CH_3$ | $C_2H_5$ | |

Tabelle 1 (Fortsetzung)

| Nr. | $R_m$ | X | Fp(°C) / IR($cm^{-1}$) |
|---|---|---|---|
| 480 | 2,6-$F_2$, 4-$CH_3$ | $C_2H_5$ | |
| 481 | 2,6-$Br_2$, 4-$CH_3$ | $C_2H_5$ | |
| 482 | 2,4-$Cl_2$, 6-$CH_3$ | $C_2H_5$ | |
| 483 | 2,4-$F_2$, 6-$CH_3$ | $C_2H_5$ | |
| 484 | 2,4-$Br_2$, 6-$CH_3$ | $C_2H_5$ | |
| 485 | 2,6-$(CH_3)_2$, 4-F | $C_2H_5$ | |
| 486 | 2,6-$(CH_3)_2$, 4-Cl | $C_2H_5$ | |
| 487 | 2,6-$(CH_3)_2$, 4-Br | $C_2H_5$ | |
| 488 | 3,5-$(CH_3)_2$, 4-F | $C_2H_5$ | |
| 489 | 3,5-$(CH_3)_2$, 4-Cl | $C_2H_5$ | |
| 490 | 3,5-$(CH_3)_2$, 4-Br | $C_2H_5$ | |
| 491 | 2,3,6-$(CH_3)_3$, 4-F | $C_2H_5$ | |
| 492 | 2,3,6-$(CH_3)_3$, 4-Cl | $C_2H_5$ | |
| 493 | 2,3,6-$(CH_3)_3$, 4-Br | $C_2H_5$ | |
| 494 | 2,4-$(CH_3)_2$, 6-F | $C_2H_5$ | |
| 495 | 2,4-$(CH_3)_2$, 6-Cl | $C_2H_5$ | |
| 496 | 2,4-$(CH_3)_2$, 6-Br | $C_2H_5$ | |
| 497 | 2-i-$C_3H_7$, 4-Cl, 5-$CH_3$ | $C_2H_5$ | |
| 498 | 2-$CH_3$, 4-Cl, 5-i-$C_3H_7$ | $C_2H_5$ | |
| 499 | 2-Cl, 3-i-$C_3H_7$ | $C_2H_5$ | |
| 500 | 2-Cl, 4-i-$C_3H_7$ | $C_2H_5$ | |
| 501 | 2-Cl, 4-$NO_2$ | $C_2H_5$ | |
| 502 | 2-$NO_2$, 4-Cl | $C_2H_5$ | |

Tabelle 1 (Fortsetzung)

| Nr. | $R_m$ | X | Fp(°C) / IR($cm^{-1}$) |
|---|---|---|---|
| 478 | 2-Br, 3,5-$(CH_3)_2$ | $C_2H_5$ | |
| 479 | 2,6-$Cl_2$, 4-$CH_3$ | $C_2H_5$ | |
| 480 | 2,6-$F_2$, 4-$CH_3$ | $C_2H_5$ | |
| 481 | 2,6-$Br_2$, 4-$CH_3$ | $C_2H_5$ | |
| 482 | 2,4-$Cl_2$, 6-$CH_3$ | $C_2H_5$ | |
| 483 | 2,4-$F_2$, 6-$CH_3$ | $C_2H_5$ | |
| 484 | 2,4-$Br_2$, 6-$CH_3$ | $C_2H_5$ | |
| 485 | 2,6-$(CH_3)_2$, 4-F | $C_2H_5$ | |
| 486 | 2,6-$(CH_3)_2$, 4-Cl | $C_2H_5$ | |
| 487 | 2,6-$(CH_3)_2$, 4-Br | $C_2H_5$ | |
| 488 | 3,5-$(CH_3)_2$, 4-F | $C_2H_5$ | |
| 489 | 3,5-$(CH_3)_2$, 4-Cl | $C_2H_5$ | |
| 490 | 3,5-$(CH_3)_2$, 4-Br | $C_2H_5$ | |
| 491 | 2,3,6-$(CH_3)_3$, 4-F | $C_2H_5$ | |
| 492 | 2,3,6-$(CH_3)_3$, 4-Cl | $C_2H_5$ | |
| 493 | 2,3,6-$(CH_3)_3$, 4-Br | $C_2H_5$ | |
| 494 | 2,4-$(CH_3)_2$, 6-F | $C_2H_5$ | |
| 495 | 2,4-$(CH_3)_2$, 6-Cl | $C_2H_5$ | |
| 496 | 2,4-$(CH_3)_2$, 6-Br | $C_2H_5$ | |
| 497 | 2-i-$C_3H_7$, 4-Cl, 5-$CH_3$ | $C_2H_5$ | |
| 498 | 2-$CH_3$, 4-Cl, 5-i-$C_3H_7$ | $C_2H_5$ | |
| 499 | 2-Cl, 3-i-$C_3H_7$ | $C_2H_5$ | |
| 500 | 2-Cl, 4-i-$C_3H_7$ | $C_2H_5$ | |

Tabelle 1 (Fortsetzung)

| Nr. | $R_m$ | X | Fp(°C) / IR($cm^{-1}$) |
|---|---|---|---|
| 501 | 2-Cl, 4-$NO_2$ | $C_2H_5$ | |
| 502 | 2-$NO_2$, 4-Cl | $C_2H_5$ | |
| 503 | 2-$OCH_3$, 5-$NO_2$ | $C_2H_5$ | |
| 504 | 2,4-$Cl_2$, 5-$NO_2$ | $C_2H_5$ | |
| 505 | 2,4-$Cl_2$, 6-$NO_2$ | $C_2H_5$ | |
| 506 | 2,6-$Cl_2$, 4-$NO_2$ | $C_2H_5$ | |
| 507 | 2,6-$Br_2$, 4-$NO_2$ | $C_2H_5$ | |
| 508 | 2,6-$J_2$, 4-$NO_2$ | $C_2H_5$ | |
| 509 | H | H | |
| 510 | 2-F | H | |
| 511 | 3-F | H | |
| 512 | 4-F | H | |
| 513 | 2,3-$F_2$ | H | |
| 514 | 2,4-$F_2$ | H | |
| 515 | 2,4,6-$F_3$ | H | |
| 516 | 2,3,4,5,6-$F_5$ | H | |
| 517 | 2-Cl | H | |
| 518 | 3-Cl | H | |
| 519 | 4-Cl | H | |
| 520 | 2,3-$Cl_2$ | H | |
| 521 | 2,4-$Cl_2$ | H | |
| 522 | 2,5-$Cl_2$ | H | |
| 523 | 2,6-$Cl_2$ | H | |

Tabelle 1 (Fortsetzung)

| Nr. | $R_m$ | X | Fp(°C) / IR($cm^{-1}$) |
|---|---|---|---|
| 524 | 3,4-$Cl_2$ | H | |
| 525 | 3,5-$Cl_2$ | H | |
| 526 | 2,3,4-$Cl_3$ | H | |
| 527 | 2,3,5-$Cl_3$ | H | |
| 528 | 2,3,6-$Cl_3$ | H | |
| 529 | 2,4,5-$Cl_3$ | H | |
| 530 | 2,4,6-$Cl_3$ | H | |
| 531 | 3,4,5-$Cl_3$ | H | |
| 532 | 2,3,4,6-$Cl_4$ | H | |
| 533 | 2,3,5,6-$Cl_4$ | H | |
| 534 | 2,3,4,5,6-$Cl_5$ | H | |
| 535 | 2-Br | H | |
| 536 | 3-Br | H | |
| 537 | 4-Br | H | |
| 538 | 2,4-$Br_2$ | H | |
| 539 | 2,5-$Br_2$ | H | |
| 540 | 2,6-$Br_2$ | H | |
| 541 | 2,4,6-$Br_3$ | H | |
| 542 | 2,3,4,5,6-$Br_5$ | H | |
| 543 | 2-J | H | |
| 544 | 3-J | H | |
| 545 | 4-J | H | |
| 546 | 2,4-$J_2$ | H | |

Tabelle 1 (Fortsetzung)

| Nr. | $R_m$ | X | Fp(°C) / IR($cm^{-1}$) |
|---|---|---|---|
| 547 | 2-Cl, 3-F | H | |
| 548 | 2-Cl, 4-F | H | |
| 549 | 2-Cl, 5-F | H | |
| 550 | 2-Cl, 6-F | H | |
| 551 | 2-Cl, 3-Br | H | |
| 552 | 2-Cl, 4-Br | H | |
| 553 | 2-Cl, 5-Br | H | |
| 554 | 2-Cl, 6-Br | H | |
| 555 | 2-Br, 3-Cl | H | |
| 556 | 2-Br, 4-Cl | H | |
| 357 | 2-Br, 5-Cl | H | |
| 558 | 2-Br, 3-F | H | |
| 559 | 2-Br, 4-F | H | |
| 560 | 2-Br, 5-F | H | |
| 561 | 2-Br, 6-F | H | |
| 562 | 2-F, 3-Cl | H | |
| 563 | 2-F, 4-Cl | H | |
| 564 | 2-F, 5-Cl | H | |
| 565 | 3-Cl, 4-F | H | |
| 566 | 3-Cl, 5-F | H | |
| 567 | 3-Cl, 4-Br | H | |
| 568 | 3-Cl, 5-Br | H | |
| 569 | 3-F, 4-Cl | H | |

Tabelle 1 (Fortsetzung)

| Nr. | $R_m$ | X | Fp(°C) / IR($cm^{-1}$) |
|---|---|---|---|
| 570 | 3-F, 4-Br | H | |
| 571 | 3-Br, 4-Cl | H | |
| 572 | 3-Br, 4-F | H | |
| 573 | 2,6-$Cl_2$, 4-Br | H | |
| 574 | 2-$CH_3$ | H | |
| 575 | 3-$CH_3$ | H | |
| 576 | 4-$CH_3$ | H | |
| 577 | 2,3-$(CH_3)_2$ | H | |
| 578 | 2,4-$(CH_3)_2$ | H | |
| 579 | 2,5-$(CH_3)_2$ | H | |
| 580 | 2,6-$(CH_3)_2$ | H | |
| 581 | 3,4-$(CH_3)_2$ | H | |
| 582 | 3,5-$(CH_3)_2$ | H | |
| 583 | 2,3,4-$(CH_3)_3$ | H | |
| 584 | 2,3,5-$(CH_3)_3$ | H | |
| 585 | 2,3,6-$(CH_3)_3$ | H | |
| 586 | 2,4,5-$(CH_3)_3$ | H | |
| 587 | 2,4,6-$(CH_3)_3$ | H | |
| 588 | 3,4,5-$(CH_3)_3$ | H | |
| 589 | 2,3,4,6-$(CH_3)_4$ | H | |
| 590 | 2,3,5,6-$(CH_3)_4$ | H | |
| 591 | 2,3,4,5,6-$(CH_3)_5$ | H | |
| 592 | 2-$C_2H_5$ | H | |

Tabelle 1 (Fortsetzung)

| Nr. | $R_m$ | X | Fp($^oC$) / IR($cm^{-1}$) |
|---|---|---|---|
| 593 | $3-C_2H_5$ | H | |
| 594 | $4-C_2H_5$ | H | |
| 595 | $2,4-(C_2H_5)_2$ | H | |
| 596 | $2,6-(C_2H_5)_2$ | H | |
| 597 | $3,5-(C_2H_5)_2$ | H | |
| 598 | $2,4,6-(C_2H_5)_3$ | H | |
| 599 | $2-n-C_3H_7$ | H | |
| 600 | $3-n-C_3H_7$ | H | |
| 601 | $4-n-C_3H_7$ | H | |
| 602 | $2-i-C_3H_7$ | H | |
| 603 | $3-i-C_3H_7$ | H | |
| 604 | $4-i-C_3H_7$ | H | |
| 605 | $2,4-(i-C_3H_7)_2$ | H | |
| 606 | $2,6-(i-C_3H_7)_2$ | H | |
| 607 | $2,4,6-(i-C_3H_7)_3$ | H | |
| 608 | $2,4,6-(i-C_3H_7)_3$ | H | |
| 609 | $2-s-C_4H_9$ | H | |
| 610 | $3-s-C_4H_9$ | H | |
| 611 | $4-s-C_4H_9$ | H | |
| 612 | $2-t-C_4H_9$ | H | |
| 613 | $3-t-C_4H_9$ | H | |
| 614 | $4-t-C_4H_9$ | H | |
| 615 | $2,3-(t-C_4H_9)_2$ | H | |

Tabelle 1 (Fortsetzung)

| Nr. | $R_m$ | X | Fp(°C) / IR($cm^{-1}$) |
|---|---|---|---|
| 616 | 2,4-$(t-C_4H_9)_2$ | H | |
| 617 | 2,5-$(t-C_4H_9)_2$ | H | |
| 618 | 2,6-$(t-C_4H_9)_2$ | H | |
| 619 | 3,5-$(t-C_4H_9)_2$ | H | |
| 620 | 2,4,6-$(t-C_4H_9)_3$ | H | |
| 621 | 4-n-$C_9H_{19}$ | H | |
| 622 | 4-n-$C_{12}H_{25}$ | H | |
| 623 | 3-n-$C_{15}H_{31}$ | H | |
| 624 | 4-(1,1,3,3,-Tetramethylbutyl) | H | |
| 625 | 4-(1,1,3,-Trimethylbutyl) | H | |
| 626 | 2-t-$C_4H_9$, 4-$CH_3$ | H | |
| 627 | 2-t-$C_4H_9$, 5-$CH_3$ | H | |
| 628 | 2,6-$(t-C_4H_9)_2$, 4-$CH_3$ | H | |
| 629 | 2-$CH_3$, 4-t-$C_4H_9$ | H | |
| 630 | 2-$CH_3$, 6-t-$C_4H_9$ | H | |
| 631 | 2-$CH_3$, 4-i-$C_3H_7$ | H | |
| 632 | 2-$CH_3$, 5-i-$C_3H_7$ | H | |
| 633 | 3-$CH_3$, 4-i-$C_3H_7$ | H | |
| 634 | 2-i-$C_3H_7$, 5-$CH_3$ | H | |
| 635 | 2,4-$(t-C_4H_9)_2$, 6-i-$C_3H_7$ | H | |
| 636 | 2-Allyl ($C_3H_5$) | H | |
| 637 | 3-Allyl | H | |
| 638 | 4-Allyl | H | |

Tabelle 1 (Fortsetzung)

| Nr. | $R_m$ | X | Fp(°C) / IR($cm^{-1}$) |
|---|---|---|---|
| 639 | $2-C_3H_5$, $6-CH_3$ | H | |
| 640 | $2-cyclo-C_6H_{11}$ | H | |
| 641 | $3-cyclo-C_6H_{11}$ | H | |
| 642 | $4-cyclo-C_6H_{11}$ | H | |
| 643 | $2,4-(cyclo-C_6H_{11})_2$, $6-CH_3$ | H | |
| 644 | $2-CH_3$, $4-cyclo-C_6H_{11}$ | H | |
| 645 | $2-CH_3$, 4-(1,1,3,3-Tetramethylbutyl) | H | |
| 646 | $2-CH_2C_6H_5$ | H | |
| 647 | $3-CH_2C_6H_5$ | H | |
| 648 | $4-CH_2C_6H_5$ | H | |
| 649 | $2-CH_2C_6H_5$, $4-CH_3$ | H | |
| 650 | $2-CH_3$, $4-CH_2C_6H_5$ | H | |
| 651 | $2-C_6H_5$ | H | |
| 652 | $3-C_6H_5$ | H | |
| 653 | $4-C_6H_5$ | H | |
| 654 | $4-(2-i-C_3H_7-C_6H_4)$ | H | |
| 655 | $4-C_6H_5$, $2,6-(CH_3)_2$ | H | |
| 656 | 2-Cl, $4-C_6H_5$ | H | |
| 657 | 2-Br, $4-C_6H_5$ | H | |
| 658 | $2-C_6H_5$, 4-Cl | H | |
| 659 | $2-C_6H_5$, 4-Br | H | |
| 660 | $2-CH_2C_6H_5$, 4-Cl | H | |
| 661 | $2-CH_2C_6H_5$, 4-Br | H | |

Tabelle 1 (Fortsetzung)

| Nr. | $R_m$ | X | Fp(°C) / IR($cm^{-1}$) |
|---|---|---|---|
| 662 | 2-Cl, 4-$CH_2C_6H_5$ | H | |
| 663 | 2-Br, 4-$CH_2C_6H_5$ | H | |
| 664 | 2-cyclo-$C_6H_{11}$, 4-Cl | H | |
| 665 | 2-cyclo-$C_6H_{11}$, 4-Br | H | |
| 666 | 2-Cl, 4-cyclo-$C_6H_{11}$ | H | |
| 667 | 2-Br, 4-cyclo-$C_6H_{11}$ | H | |
| 668 | 2-$OCH_3$ | H | |
| 669 | 3-$OCH_3$ | H | |
| 670 | 4-$OCH_3$ | H | |
| 671 | 2,4-$(OCH_3)_2$ | H | |
| 672 | 2-$OC_2H_5$ | H | |
| 673 | 3-$OC_2H_5$ | H | |
| 674 | 4-$OC_2H_5$ | H | |
| 675 | 2-$OCH_2C_6H_5$ | H | |
| 676 | 3-$OCH_2C_6H_5$ | H | |
| 677 | 4-$OCH_2C_6H_5$ | H | |
| 678 | 2-O-t-$C_4H_9$ | H | |
| 679 | 3-O-t-$C_4H_9$ | H | |
| 680 | 4-O-t-$C_4H_9$ | H | |
| 681 | 2-$OC_6H_5$ | H | |
| 682 | 3-$OC_6H_5$ | H | |
| 683 | 4-$OC_6H_5$ | H | |
| 684 | 2-$CF_3$ | H | |

Tabelle 1 (Fortsetzung)

| Nr. | $R_m$ | X | Fp($^oC$) / IR($cm^{-1}$) |
|---|---|---|---|
| 685 | 3-$CF_3$ | H | |
| 686 | 4-$CF_3$ | H | |
| 687 | 2-$OCF_3$ | H | |
| 688 | 3-$OCF_3$ | H | |
| 689 | 4-$OCF_3$ | H | |
| 690 | 3-$OCH_2CHF_2$ | H | |
| 691 | 3-$OCF_2CHF_2$ | H | |
| 692 | 3-$OC_2F_5$ | H | |
| 693 | 2-$NO_2$ | H | |
| 694 | 3-$NO_2$ | H | |
| 695 | 4-$NO_2$ | H | |
| 696 | 2-CN | H | |
| 697 | 3-CN | H | |
| 698 | 4-CN | H | |
| 699 | 2-$CH_3$, 3-Cl | H | |
| 700 | 2-$CH_3$, 4-Cl | H | |
| 701 | 2-$CH_3$, 5-Cl | H | |
| 702 | 2-$CH_3$, 6-Cl | H | |
| 703 | 2-$CH_3$, 3-F | H | |
| 704 | 2-$CH_3$, 4-F | H | |
| 705 | 2-$CH_3$, 5-F | H | |
| 706 | 2-$CH_3$, 6-F | H | |
| 707 | 2-$CH_3$, 3-Br | H | |

Tabelle 1 (Fortsetzung)

| Nr. | $R_m$ | X | Fp(°C) / IR($cm^{-1}$) |
|---|---|---|---|
| 708 | 2-$CH_3$, 4-Br | H | |
| 709 | 2-$CH_3$, 5-Br | H | |
| 710 | 2-$CH_3$, 6-Br | H | |
| 711 | 2-Cl, 3-$CH_3$ | H | |
| 712 | 2-Cl, 4-$CH_3$ | H | |
| 713 | 2-Cl, 5-$CH_3$ | H | |
| 714 | 2-F, 3-$CH_3$ | H | |
| 715 | 2-F, 4-$CH_3$ | H | |
| 716 | 2-F, 5-$CH_3$ | H | |
| 717 | 2-Br, 3-$CH_3$ | H | |
| 718 | 2-Br, 4-$CH_3$ | H | |
| 719 | 2-Br, 5-$CH_3$ | H | |
| 720 | 3-$CH_3$, 4-Cl | H | |
| 721 | 3-$CH_3$, 5-Cl | H | |
| 722 | 3-$CH_3$, 4-F | H | |
| 723 | 3-$CH_3$, 5-F | H | |
| 724 | 3-$CH_3$, 4-Br | H | |
| 725 | 3-$CH_3$, 5-Br | H | |
| 726 | 3-F, 4-$CH_3$ | H | |
| 727 | 3-Cl, 4-$CH_3$ | H | |
| 728 | 3-Br, 4-$CH_3$ | H | |
| 729 | 2-Cl, 4,5-$(CH_3)_2$ | H | |
| 730 | 2-Br, 4,5-$(CH_3)_2$ | [illegible] | |

Tabelle 1 (Fortsetzung)

| Nr. | $R_m$ | X | Fp($^oC$) / IR($cm^{-1}$) |
|---|---|---|---|
| 731 | 2-Cl, 3,5-$(CH_3)_2$ | H | |
| 732 | 2-Br, 3,5-$(CH_3)_2$ | H | |
| 733 | 2,6-$Cl_2$, 4-$CH_3$ | H | |
| 734 | 2,6-$F_2$, 4-$CH_3$ | H | |
| 735 | 2,6-$Br_2$, 4-$CH_3$ | H | |
| 736 | 2,4-$Cl_2$, 6-$CH_3$ | H | |
| 737 | 2,4-$F_2$, 6-$CH_3$ | H | |
| 738 | 2,4-$Br_2$, 6-$CH_3$ | H | |
| 739 | 2,6-$(CH_3)_2$, 4-F | H | |
| 740 | 2,6-$(CH_3)_2$, 4-Cl | H | |
| 741 | 2,6-$(CH_3)_2$, 4-Br | H | |
| 742 | 3,5-$(CH_3)_2$, 4-F | H | |
| 743 | 3,5-$(CH_3)_2$, 4-Cl | H | |
| 744 | 3,5-$(CH_3)_2$, 4-Br | H | |
| 745 | 2,3,6-$(CH_3)_3$, 4-F | H | |
| 746 | 2,3,6-$(CH_3)_3$, 4-Cl | H | |
| 747 | 2,3,6-$(CH_3)_3$, 4-Br | H | |
| 748 | 2,4-$(CH_3)_2$, 6-F | H | |
| 749 | 2,4-$(CH_3)_2$, 6-Cl | H | |
| 750 | 2,4-$(CH_3)_2$, 6-Br | H | |
| 751 | 2-i-$C_3H_7$, 4-Cl, 5-$CH_3$ | H | |
| 752 | 2-$CH_3$, 4-Cl, 5-i-$C_3H_7$ | H | |
| 753 | 2-Cl, 3-i-$C_3H_7$ | H | |

Tabelle 1 (Fortsetzung)

| Nr. | $R_m$ | X | Fp(°C) / IR($cm^{-1}$) |
|---|---|---|---|
| 754 | 2-Cl, 4-i-$C_3H_7$ | H | |
| 755 | 2-Cl, 4-$NO_2$ | H | |
| 756 | 2-$NO_2$, 4-Cl | H | |
| 757 | 2-$OCH_3$, 5-$NO_2$ | H | |
| 758 | 2,4-$Cl_2$, 5-$NO_2$ | H | |
| 759 | 2,4-$Cl_2$, 6-$NO_2$ | H | |
| 760 | 2,6-$Cl_2$, 4-$NO_2$ | H | |
| 761 | 2,6-$Br_2$, 4-$NO_2$ | H | |
| 762 | 2,6-$J_2$, 4-$NO_2$ | H | |
| 763 | 2-$CH_3$ | n-$C_3H_7$ | |
| 764 | 2-$CH_3$ | i-$C_3H_7$ | |
| 765 | 2-$CH_3$ | cyclo-$C_3H_5$ | |
| 766 | 2-$CH_3$ | cyclo-$C_3H_5$ | |
| 767 | 2-$CH_3$ | cyclo-$C_5H_9$ | |
| 768 | 2-$CH_3$ | cyclo-$C_6H_{11}$ | |
| 769 | 2-$CH_3$ | n-$C_4H_9$ | |
| 770 | 2-$CH_3$ | i-$C_4H_9$ | |
| 771 | 2-$CH_3$ | s-$C_4H_9$ | |
| 772 | 2-$CH_3$ | t-$C_4H_9$ | |
| 773 | 2-$CH_3$ | n-$C_5H_{11}$ | |
| 774 | 2-$CH_3$ | n-$C_6H_{13}$ | |
| 775 | H | n-$C_3H_7$ | |
| 776 | H | i-$C_3H_7$ | |

Tabelle 1 (Fortsetzung)

| Nr. | $R_m$ | X | Fp(°C) / IR($cm^{-1}$) |
|---|---|---|---|
| 777 | H | cyclo-$C_3H_5$ | |
| 778 | H | cyclo-$C_3H_5$ | |
| 779 | H | cyclo-$C_5H_9$ | |
| 780 | H | cyclo-$C_6H_{11}$ | |
| 781 | H | n-$C_4H_9$ | |
| 782 | H | n-$C_5H_{11}$ | |
| 783 | H | n-$C_6H_{13}$ | |
| 784 | *1) | $CH_3$ | |
| 785 | *1) | $C_2H_5$ | |
| 786 | *1) | H | |
| 787 | *2) | $CH_3$ | |
| 788 | *2) | $C_2H_5$ | |
| 789 | *2) | H | |

*1) 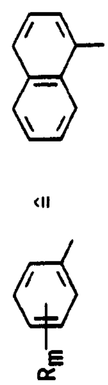

*2) 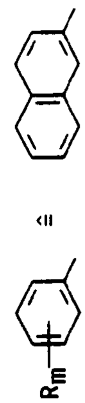

Die Anilinderivate I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse, wie Gurken, Bohnen und Kürbisgewächse.

Die Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,

Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze. Es werden die Pilze oder die vor Pilzbefall zu schützenden Pflanzen, Saatgüter, Materialien oder der Erdboden mit einer fungizid wirksamen Menge des Wirkstoffs behandelt.

Die Verbindungen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken der wirksamen Substanz Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die Verbindungen I können auch im Materialschutz (Holzschutz) eingesetzt werden, z.B. gegen Paecilomyces variotii. Sei der Saatgutbehandlung werden im allgemeinen Wirkstoffmengen von 0,001 bis 50 g, vorzugsweise 0,01 bis 10 g, je Kilogramm Saatgut benötigt.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1 mit 10 Gew.-Teilen N-Methyl-$\alpha$-pyrrolidon und erhält eine Lösung, die zur Anwendung in Form kleinster Tropfen geeignet ist.

II. 20 Gew.-Teile der Verbindung Nr. 1 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 4 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 68 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 4 werden mit 3 Gew.-Teilen des Natriumsalzes der Diisobutylnaphthalin-$\alpha$-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 68 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 4 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 1 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen fungiziden Mittel können in diesen Anwendungsformen auch zusammen mit anderen Wirkstoffen vorliegen, wie z.B. Herbiziden, Insektiziden, Wachstumsregulatoren und anderen Fungiziden, oder auch mit Düngemitteln vermischt und ausgebracht werden. Beim Vermischen mit anderen Fungiziden erhält man dabei in vielen Fällen eine Vergrößerung des fungiziden Wirkungsspektrums.

Anwendungsbeispiel

Beispiel 1

Wirksamkeit gegen Pyrenophora teres (Netzfleckenkrankheit der Gerste)

Gerstenkeimlinge der Sorte "Igri" wurden im Zweiblattstadium mit wäßrigen Suspensionen, die 80 % Wirkstoff und 20 % Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in eine Klimakammer mit hoher Luftfeuchtigkeit bei 18°C gestellt. Anschließend wurden die Pflanzen im Gewächshaus bei 20 bis 22°C und 70 % relativer Luftfeuchtigkeit für weitere 5 Tage kultiviert. Dann wurde das Ausmaß der Symptomentwicklung ermittelt.

Vergleichswirkstoff ist 2-Phenoxymethyl-phenyl-glyoxylsäuremethylester-O-methyloxim, das aus DE-A-3 623 921 bekannt ist (Verbindung Nr. 124).

Das Ergebnis der Prüfung zeigt, daß beispielsweise die erfindungsgemäße Verbindung Nr. 1 eine dem bekannten Wirkstoff deutlich überlegene fungizide Wirkung hat.

**Patentansprüche**

1. Anilinderivate der Formel I

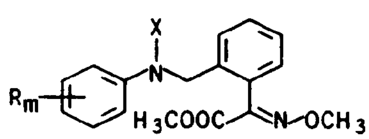

(I)

in der

R

Wasserstoff, Halogen, Cyano, Nitro, $C_1$-$C_{15}$-Alkyl, $C_3$-$C_6$-Cycloalkyl, $C_3$-$C_6$-Alkenyl, $C_1$-$C_4$-Alkoxy, $C_1$-$C_2$-Halogenalkyl, $C_1$-$C_2$-Halogenalkoxy, gegebenenfalls substituiertes Phenyl, gegebenenfalls substituiertes Phenoxy, gegebenenfalls substituiertes Benzyl oder gegebenenfalls substituiertes Benzyloxy,

m

eine ganze Zahl von 1 bis 5 oder die Gruppe

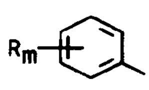

α-Naphthyl oder β-Naphthyl und

X

Wasserstoff, $C_1$-$C_6$-Alkyl oder $C_3$-$C_6$-Cycloalkyl bedeuten,

sowie ihre pflanzenverträglichen Säureadditionssalze und Metallkomplexverbindungen.

2. Anilinderivat der Formel I gemäß Anspruch 1, dadurch gekennzeichnet, daß $R_m$ Wasserstoff und X Methyl

bedeutet.

3. Anilinderivat der Formel I gemäß Anspruch 1, dadurch gekennzeichnet, daß $R_m$ 4-Fluor und X Methyl bedeutet.

4. Anilinderivat der Formel I in Anspruch 1, dadurch gekennzeichnet, daß $R_m$ 4-Methyl und X Methyl bedeutet.

5. Verfahren zur Herstellung von Anilinderivaten der Formel I gemäß Anspruch 1, dadurch gekennzeichnet, daß man ein Benzylhalogenid der allgemeinen Formel II

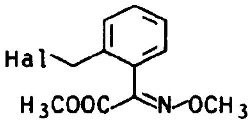

(II),

in der Hal für Chlor, Brom oder Jod steht, mit einem Anilin der Formel III

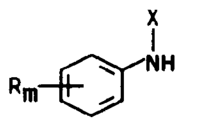

(III),

in der
R, m und X die in Anspruch 1 genannten Bedeutungen haben, umsetzt.

6. Fungizid, enthaltend einen inerten Trägerstoff und eine fungizid wirksame Menge eines Anilinderivats der Formel I

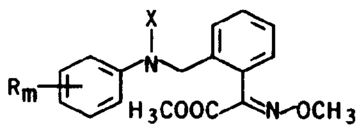

(I)

in der
R
Wasserstoff, Halogen, Cyano, Nitro, $C_1$-$C_{15}$-Alkyl, $C_3$-$C_6$-Cycloalkyl, $C_3$-$C_6$-Alkenyl, $C_1$-$C_4$-Alkoxy, $C_1$-$C_2$-Halogenalkyl, $C_1$-$C_2$-Halogenalkoxy, gegebenenfalls substituiertes Phenyl, gegebenenfalls substituiertes Phenoxy, gegebenenfalls substituiertes Benzyl oder gegebenenfalls substituiertes Benzyloxy,
m
eine ganze Zahl von 1 bis 5 oder die Gruppe

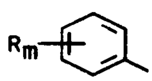

α-Naphthyl oder β-Naphthyl und
X
Wasserstoff, $C_1$-$C_6$-Alkyl oder $C_3$-$C_6$-Cycloalkyl bedeuten,
oder ihres pflanzenverträglichen Säureadditionssalzes oder ihrer Metallkomplexverbindung.

7. Verfahren zur Bekämpfung von Pilzen, dadurch gekennzeichnet, daß man die Pilze oder die vom Pilzbefall bedrohten Materialien, Pflanzen, Saatgüter oder den Erdboden mit einer fungizid wirksamen Menge eines Anilinderivats der Formel I

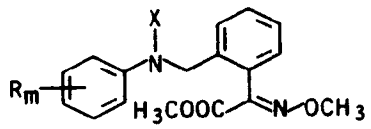

(I)

in der

R

Wasserstoff, Halogen, Cyano, Nitro, $C_1$-$C_{15}$-Alkyl, $C_3$-$C_6$-Cycloalkyl, $C_3$-$C_6$-Alkenyl, $C_1$-$C_4$-Alkoxy, $C_1$-$C_2$-Halogenalkyl, $C_1$-$C_2$-Halogenalkoxy, gegebenenfalls substituiertes Phenyl, gegebenenfalls substituiertes Phenoxy, gegebenenfalls substituiertes Benzyl oder gegebenenfalls substituiertes Benzyloxy,

m

eine ganze Zahl von 1 bis 5 oder die Gruppe

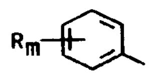

α-Naphthyl oder β-Naphthyl und

X

Wasserstoff, $C_1$-$C_6$-Alkyl oder $C_3$-$C_6$-Cycloalkyl bedeuten,

oder ihres pflanzenverträglichen Säureadditionssalzes oder ihrer Metallkomplexverbindung behandelt.

## Claims

1. An aniline derivative of the formula I

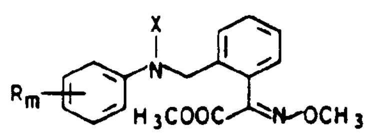

(I)

where

R is hydrogen, halogen, cyano, nitro, $C_1$-$C_{15}$-alkyl, $C_3$-$C_6$-cycloalkyl, $C_3$-$C_6$-alkenyl, $C_1$-$C_4$-alkoxy, $C_1$- or $C_2$-haloalkyl, $C_1$- or $C_2$-haloalkoxy, substituted or unsubstituted phenyl, substituted or unsubstituted phenoxy, substituted or unsubstituted benzyl or substituted or unsubstituted benzyloxy,

m is an integer of from 1 to 5 or the group

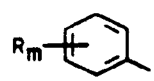

α-naphthyl or β-naphthyl and

X is hydrogen, $C_1$-$C_6$-alkyl or $C_3$-$C_6$-cycloalkyl,

or a plant-tolerated acid addition salt or metal complex thereof.

2. An aniline derivative of the formula I as claimed in claim 1, wherein $R_m$ is hydrogen and X is methyl.

3. An aniline derivative of the formula I as claimed in claim 1, wherein $R_m$ is 4-fluorine and X is methyl.

4. An aniline derivative of the formula I as claimed in claim 1, wherein $R_m$ is 4-methyl and X is methyl.

5. A process for preparing an aniline derivative of the formula I as claimed in claim 1, which comprises reacting a benzyl halide of the formula II

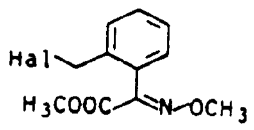

(II)

where Hal is fluorine, bromine or iodine, with an aniline of the formula III

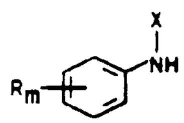

(III)

where R, m and X have the meanings stated in claim 1.

**6.** A fungicide containing an inert carrier and a fungicidally effective amount of an aniline derivative of the formula I

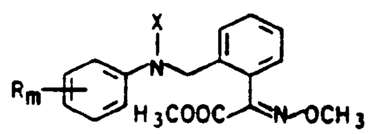

(I)

where
R is hydrogen, halogen, cyano, nitro, $C_1$-$C_{15}$-alkyl, $C_3$-$C_6$-cycloalkyl, $C_3$-$C_6$-alkenyl, $C_1$-$C_4$-alkoxy, $C_1$- or $C_2$-haloalkyl, $C_1$- or $C_2$-haloalkoxy, substituted or unsubstituted phenyl, substituted or unsubstituted phenoxy, substituted or unsubstituted benzyl or substituted or unsubstituted benzyloxy,
m is an integer of from 1 to 5 or the group

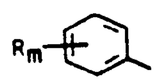

α-naphthyl or β-naphthyl and
X is hydrogen, $C_1$-$C_6$-alkyl or $C_3$-$C_6$-cycloalkyl,
or a plant-tolerated acid addition salt or metal complex thereof.

**7.** A method for controlling fungi, wherein the fungi or the materials, plants, seed or the soil threatened by fungal attack are (is) treated with a fungicidally effective amount of an aniline derivative of the formula I

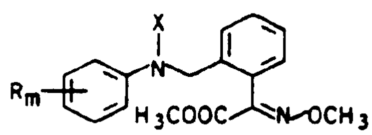

(I)

where
R is hydrogen, halogen, cyano, nitro, $C_1$-$C_{15}$-alkyl, $C_3$-$C_6$-cycloalkyl, $C_3$-$C_6$-alkenyl, $C_1$-$C_4$-alkoxy, $C_1$- or $C_2$-haloalkyl, $C_1$- or $C_2$-haloalkoxy, substituted or unsubstituted phenyl, substituted or unsubstituted phenoxy, substituted or unsubstituted benzyl or substituted or unsubstituted benzyloxy,
m is an integer of from 1 to 5 or the group

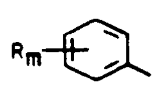

α-naphthyl or β-naphthyl and
X is hydrogen, $C_1$-$C_6$-alkyl or $C_3$-$C_6$-cycloalkyl,
or a plant-tolerated acid addition salt or metal complex thereof.

## Revendications

**1.** Dérivés d'aniline de formule I

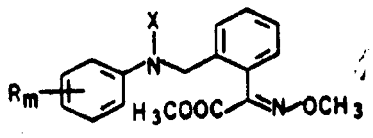

(I),

dans laquelle

R représente un atome d'hydrogène, un halogène, un cyano, un nitro, un alkyle en $C_1$-$C_{15}$, un cycloalkyle en $C_3C_6$, un alcényle en $C_3$-$C_6$, un alkoxy en $C_1$-$C_4$, un halogénoalkyle en $C_1$-$C_2$, un halogénalkoxy en $C_1$-$C_2$, le cas échéant un phényle substitué, le cas échéant un phénoxy substitué, le cas écheant un benzyle substitué, ou le cas échéant un benzyloxy substitué,

m un nombre entier de 1 à 5 ou bien le groupement

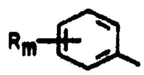

alpha-naphtyle ou béta-naphtyle et

X un atome d'hydrogène, un alkyle en $C_1$-$C_6$, un cycloalkyle en $C_3$-$C_6$,

ainsi que leurs sels d'addition acides et leurs composés métalliques complexés, qui sont tolérés par les plantes.

**2.** Dérivé d'aniline de formule I selon la revendication 1, caractérisé en ce que $R_m$ représente un atome d'hydrogène et X, un groupement méthyle.

**3.** Dérivé d'aniline de formule I selon la revendication 1, caractérisé en ce que $R_m$ représente un fluor-4 et X, un groupement méthyle.

**4.** Dérivé d'aniline de formule I selon la revendication 1, caractérisé en ce que $R_m$ représente un groupement méthyl-4 et X, un groupement méthyle.

**5.** Procédé pour la fabrication de dérivés d'aniline de formule I selon la revendication 1, caractérisé en ce que l'on fait réagir un halogénure de benzyle de formule générale II

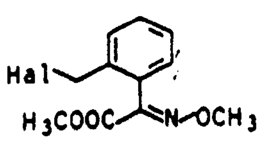

(II),

dans laquelle Hal représente un atome de chlore, de brome ou d'iode, avec une aniline de formule III

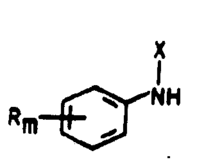

(III),

dans laquelle

R, m et X conservent la signification fixée dans la revendication 1.

6. Fongicide comprenant un composé porteur inerte et une dose active de fongicide constitué d'un dérivé d'aniline de formule I

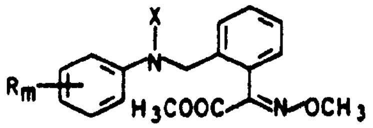

(I)

dans laquelle

R représente un atome d'hydrogène, un halogène, un cyano, un nitro, un alkyle en $C_1$-$C_{15}$, un cycloalkyle en $C_3$-$C_6$, un alcényle en $C_3$-$C_6$, un alkoxy en $C_1$-$C_4$, un halogénoalkyle en $C_1$-$C_2$, un halogénoalkoxy en $C_1$-$C_2$, le cas échéant un phényle substitué, le cas échéant un phénoxy substitué, le cas échéant un benzyle substitué, ou le cas échéant un benzyloxy substitué,

m un nombre entier de 1 à 5 ou bien le groupement

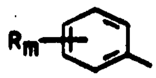

alpha-naphtyle ou béta-naphtyle et

X un atome d'hydrogène, un alkyle en $C_1$-$C_6$ ou un cycloalkyle en $C_3$-$C_6$, ou son sel d'addition acide et son composé métallique complexé, qui sont tolérés par les plantes.

7. Procédé de lutte contre les champignons, caractérisé en ce que l'on traite les champignons ou les matières, les plantes, les semences ou le sol menacés d'une attaque par les champignons par une dose active de fongicide constitué d'un dérivé d'aniline de formule I

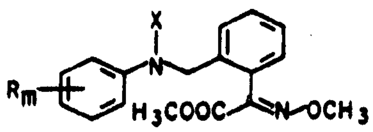

(I)

dans laquelle

R représente un atome d'hydrogène, un halogène, un cyano, un nitro, un alkyle en $C_1$-$C_{15}$, un cycloalkyle en $C_3$-$C_6$, un alcényle en $C_3$-$C_6$, un alkoxy en $C_1$-$C_4$, un halogénoalkyle en $C_1$-$C_2$, un halogénoalkoxy en $C_1$-$C_2$, le cas échéant un phényle substitué, le cas échéant un phénoxy substitué, le cas échéant un benzyle substitué, ou le cas échéant un benzyloxy substitué,

m un nombre entier de 1 à 5 ou bien le groupement

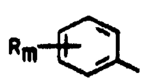

alpha-naphtyle ou béta-naphtyle et

X un atome d'hydrogène, un alkyle en $C_1$-$C_6$, un cycloalkyle en $C_3$-$C_6$,
ou son sel d'addition acide et son composé métallique complexé, qui sont tolérés par les plantes.